# EUROPÄISCHE PATENTANMELDUNG

(11) **EP 0 581 062 A1**
(43) Veröffentlichungstag der Anmeldung: **02.02.1994**
(21) Anmeldenummer: 93110752.8
(22) Anmeldetag: 06.07.1993
(51) Int. Cl.: A61K 31/41

(54) **Verwendung von 1,2,5-Oxadiazol-2-oxiden zur Herstellung eines Arzneimittels zur Behandlung erektiler Dysfunktionen**

(30) Priorität: 20.07.1992 DE 4223800
(71) Anmelder: CASSELLA Aktiengesellschaft, D-60386 Frankfurt (DE)
(72) Erfinder: Schönafinger, Karl, Dr., D-63755 Alzenau (DE); Brendel, Joachim, Dr., D-61118 Bad Vilbel (DE); Stief, Christian, Dr., D-30966 Hemmingen 1 (DE)
(74) Vertreter: Muley, Ralf, Dr.

(57) **Zusammenfassung**

Die vorliegende Erfindung betrifft die Verwendung von 1.2.5-Oxadiazol-2-oxiden der allgemeinen Formel I 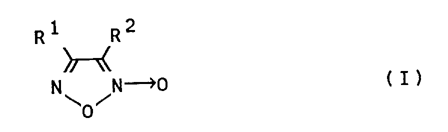
oder eines pharmakologisch verträglichen Salzes davon, wobei R¹ und R² wie in Anspruch 1 angegeben definiert sind, zur Behandlung erektiler Dysfunktionen.

## Beschreibung

Die Vorliegende Erfindung betrifft die Verwendung von 1.2.5-Oxadiazol-2-oxiden sowie pharmakologisch verträgliche Salze davon zur Behandlung erektiler Dysfunktionen.

Etwa 5 % der Männer im 40. Lebensjahr und 20 % im 60. Lebensjahr leiden an einer erektilen Dysfunktion. Diese Störungen bedürfen einer medizinischen Behandlung, da insbesondere Patienten mit chronischer erektiler Dysfunktion in Ihrer Sexualität und Persönlichkeit verunsichert und als krank anzusehen sind.

Zur Behandlung von bis in die siebziger Jahre überwiegend auf psychische Ursachen zurückgeführten Potenzstörungen wurden neben psychotherapeutischen Maßnahmen Testosterone oder Aphrodisiaka von umstrittenem Wert eingesetzt. Erst durch die Erforschung der Physiologie des Erektionsablaufs wurde festgestellt, daß bei mehr als 60 % der Patienten organische Ursachen die Erektionsstörung bewirken, wobei autonome Efferenzen aus dem parasympathischen Anteil des Sakralmarkes, Neurotransmitter, Dilatation der Penisarterien, Relaxation der cavernösen Räume und Konstriktion der Venen eine Rolle spielen. In über 70 % der Fälle sind vaskuläre Faktoren ursächlich beteiligt, wie pathologische arterielle Blutversorgungen oder abnorm gesteigerter venöser Abfluß aus den kavernosen Räumen. Neurogene Störungen sind zu etwa 20 % beteiligt.

Die orale Therapie dieser organisch bedingten Dysfunktionen mit vasoaktiven Substanzen wie Yohimbin, Phenoxybenzamin, Terbutalin, Bethanechol, Levodopa, Verapamil oder Theophyllin erwies sich als erfolglos. Neben der Anwendung von Prothesenimplantationen oder Revaskularisierungsoperationen erwies sich eine intrakavernöse Injektion von Papaverin (Virag, Lancet, 2, 938, 1982), dem α-Rezeptorenblocker Phenoxybenzamin (Brindley, Br. J. Psychiatr., 143, 332, 1983) und eine Kombination von Papaverin und dem α-Rezeptorenblocker Phentolamin (Stief, Urologe A, 25, 63, 1986) als erfolgreich. Letztere Therapiemethode läßt sich vom Patienten selbständig durchführen und wird auch als Schwellkörper-Autoinjektionstherapie (SKAT) bezeichnet.

Nachteilig erwiesen sich jedoch eine teilweise unerwünscht verlängerte Erektion mit der Gefahr des Priapismus bei der Verwendung von Papaverin, unerwünschte Schmerzhaftigkeit bei der Anwendung von Phenoxybenzamin sowie eine mögliche Cancerogenität dieser Verbindung.

Im Tierexperiment (Cynomolgus) wurden zudem bei 1 - 2 Intracavernös-Injektionen Papaverin wöchentlich über 12 Monate ausgedehnte Fibrosierungen weiter Teile des Schwellkörpers festgestellt, was beim Menschen zu äußerst negativen Langzeitergebnissen führen würde, da bei einer Fibroisierung des corpus cavernosum keine Erektion mehr erfolgen kann.

Die Anwendung von Acetylcholin ist bei nur kurzzeitiger Erektion mit starken systemischen Nebenwirkungen verbunden und die Injektion von Prostaglandin E₁ wird von den Patienten wegen zu großer Schmerzhaftigkeit abgelehnt.

Aufgabe der Erfindung war daher die Entwicklung und Herstellung von Arzneimitteln zur Behandlung von neurogenen, arteriellen, neurotransmitterbedingten, myopathischen, venösen oder psychogenen erektilen Dysfunktionen ohne die genannten Nebenwirkungen.

Überraschenderweise wurde nun gefunden, daß diese Aufgabe durch 1.2.5-Oxadiazol-2-oxide gelöst werden kann. 1.2.5-Oxadiazol-2-oxide sind bereits bekannt. Neben ihren vasodilatierenden sind auch ihre antiaggregatorischen Wirkungen auf Blutplättchen beschrieben (EP-A 38 438, EP-A 54 872, EP-A 54 873, sowie Biochem. Pharmacol. 43, 1281-1288 (1992)).

Die vorliegende Erfindung betrifft somit die Verwendung von 1.2.5-Oxadiazol-2-oxiden der allgemeinen Formel I 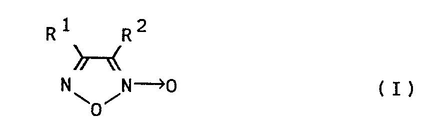
oder eines pharmakologisch verträglichen Salzes davon, wobei R¹ und R² unabhängig voneinander
a) 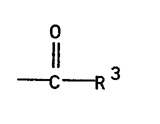 bedeuten, worin
   R³ Amino, (C₁-C₆)-Alkylamino, Di-(C₁-C₆)-alkylamino, (C₄-C₇)-Cycloalkylamino, -NHNH₂, (C₆-C₁₀)-Arylamino, -NH-(CH₂)ₙ-OR⁴,
   -NH-(CH₂)ₙ-COR⁴, -NH-(CH₂)ₙCOOR⁴-, NH-(CH₂)ₙNR⁴R^{4'}, -NH-(CH₂)n CONR⁴R^{4'}, -NH-CH(Alk)-COOR⁴, -NH-CH(Alk)--CONR⁴R^{4'}, -NH-(CH₂)ₙ-(C₆-₁₀)-Aryl, (C₁-C₆)-Alkyl, (C₆-C₁₀)-Aryl, Hydroxy,(C₁-C₆)-Alkoxy, (C₆-C₁₀)-Aryloxy oder (C₆-C₁₀)-Aryl-(C₁-C₆)-alkyloxy ist und n eine ganze Zahl von 1 bis 4, R⁴ und R^{4'}, unabhängig voneinander Wasserstoff, (C₁-C₆)-Alkyl, (C₄-C₇)-Cycloalkyl, Benzyl oder Phenethyl, Alk (C₁-C₆)-Alkyl und X -(CH₂)₂-0-(CH₂)₂-, -(CH₂)₂-N(CH₃)-(CH₂)₂-, -(CH₂)₄- oder -(CH₂)₅-bedeuten;
b) -OR⁵ bedeuten, worin
   R⁵ (C₁-C₆)-Alkyl, (C₄-C₇)-Cycloalkyl, (C₂-C₇)-Alkenyl, (C₇-C₁₀)-Aralkyl, -(CH₂)ₘ-Het, oder -(CH₂)ₘ-R⁶ ist und Het für einen Heterocylus, R⁶ für Hydroxy, (C₁-C₆)-Alkoxy, (C₁-C₆)-Alkylamino, Di-(C₁-C₆)-alkylamino oder N-(C₁-C₆)-Alkyl-N-(C₇-C₁₀)-aralkyl und m für 0, 1, 2, 3 oder 4 steht;
c) - S(O)ₚ-R⁷ bedeuten, worin
   p 0,1 oder 2 und
   R⁷ (C₁-C₆)-Alkyl, (C₄-C₇)-Cycloalkyl, (C₆-C₁₀)-Aryl, (C₇-C₁₀)-Aralkyl, (C₂-C₇)-Alkenyl, -(CH₂)ₘ-R⁶, -(CH₂)ₘ-CO-R⁸ oder -CH₂-CH(NHR⁹)COR⁸ ist, wobei m und R⁶ wie oben unter b) angegeben definiert sind und R⁸ Hydroxy, Amino, (C₁-C₆)-Alkylamino, Di-(C₁-C₆)-alkylamino oder -OR^{7'} wobei R^{7'} mit Ausnahme von -(CH₂)ₘ-CO-R⁸ wie R⁷ definiert ist und R⁹ Wasserstoff, (C₁-C₆)-Alkyl oder
   (C₂-C₇)-Alkylcarbonyl bedeuten;
d) 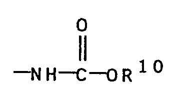 bedeuten, worin
   R¹⁰ (C₁-C₆)-Alkyl, (C₆-C₁₀)-Aryl oder (C₆-C₁₀)-Aryl-(C₁-C₆)-Alkyl ist;
e) (C₁-C₆)-Alkyl, (C₄-C₇)-Cycloalkyl, (C₁-C₆)-Alkylamino, Di-(C₁-C₆)-alkylamino, (C₆-C₁₀)-Aryl, das gegebenenfalls durch (C₁-C₆)-Alkyl, (C₁-C₆)-Alkoxy, Amino, (C₁-C₆)-Alkylamino, Di-(C₁-C₆)-Alkylamino, -COO(C₁-C₆)-Alkyl, Fluor, Chlor, Brom, Nitro oder Trifluormethyl ein-, zwei-oder dreifach substituiert ist, (C₆-C₁₀)-Aryl-(C₁-C₆)Alkyl, Heteroaryl, -(CH₂)n-COR¹¹ bedeutet, wobei n wie oben unter a) angegeben definiert ist und R¹¹ für (C₁-C₆)-Alkoxy, Amino, (C₁-C₆)-Alkylamino oder Di-(C₁-C₆)-alkylamino steht;
   wobei, falls einer der Reste R¹ und R² Pyridyl, Phenyl oder substituiertes Phenyl bedeutet, der andere nicht für ein Amid stehen kann und wobei, falls einer der Reste R¹ und R² Phenyl bedeutet, der andere nicht für einen der unter b) und c) genannten Reste stehen kann, zur Behandlung von erektilen Dysfunktionen.

(C₁-C₆)-Alkyl kann geradkettig oder verzweigt sein und bedeutet beispielsweise Methyl, Ethyl, n-Propyl, i-Propyl, n-Butyl, i-Butyl, sek.Butyl, tert. Butyl, Pentyl oder Hexyl. Entsprechendes gilt für analoge Reste, wie Alkoxy, Alkylamino usw.
(C₄-C₇)-Cycloalkyl bedeutet bevorzugt Cyclopentyl oder Cyclohexyl.

(C₂-C₇)-Alkenyl bedeutet bevorzugt Vinyl oder Allyl. (C₆-C₁₀)-Aryl bedeutet bevorzugt Phenyl.
(C₆-C₁₀)-Aryl-(C₁-C₆)-alkyl bedeutet bevorzugt Benzyl oder Phenylethyl.
(C₂-C₇)-Alkylcarbonyl ist bevorzugt Acetyl oder Propionyl.

Ein für Het stehender Heterocyclus ist bevorzugt 5-bis 7-gliedrig und kann aliphatisch oder aromatisch sein. Darüberhinaus kann er auch substituiert sein. Als Heteroglieder enthält er insbesondere 〉O, 〉S oder 〉NR⁴. Sofern der Heterocyclus 2 Heteroglieder aufweist, können diese gleich oder verschieden sein. Ein Stickstoff enthaltender Heterocyclus kann auch über das Hetero-N-atom gebunden sein; er kann dann neben dem ersten, die Bindung vermittelnden Stickstoffatom noch ein beliebiges der oben genannten Heteroglieder enthalten. Beispiele für derartige, über ein Hetero-N-atom gebundene heterocyclische Reste sind der N-Pyrrolidinrest oder der N-Thiomorpholinrest.

Aromatische heterocyclische Reste sind solche, die aufgrund einer Konjugation von Doppelbindungen, gegebenenfalls mit freien Elektronenpaaren, innerhalb des Ringes mesomere Grenzstrukturen ausbilden können, wie z. B. der Thienylrest oder der Pyrazolylrest. Aliphatische heterocyclische Reste enthalten nur isolierte oder gar keine Doppelbindungen, wie z. B. der Pyrrolidinrest, der Piperidinrest, der Morpholinrest oder der Perhydrothiazepinrest. Von den zwei Heteroglieder enthaltenden Heterocyclen sind solche bevorzugt, die mindestens ein Stickstoff enthalten.

Beispiele für Heterocyclen, von denen sich die Reste Het ableiten, sind: Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und thiazepin.

Besonders bevorzugte Reste Het leiten sich ab von Pyrrol, Pyrrolidin, Imidazol, Thiazol, Thiazolidin, Perhydrothiazepin und Pyridin.

Bei den Heterogliedern der Formel 〉N-R⁴ steht R⁴ für Wasserstoff, Alkyl mit 1 bis 4, vorzugsweise 1 oder 2, C-Atomen oder Alkoxycarbonyl mit 1 bis 4 C-Atomen in der Alkoxygruppe.

Die heterocyclischen Reste können auch an einem der Ringkohlenstoffatome einen Substituenten wie z. B. eine Carboxylgruppe, eine Formylgruppe, Alkoxycarbonyl mit 1 bis 4, vorzugsweise 1 oder 2, C-Atomen in der Alkkoxygruppe oder, bevorzugt, eine Alkylgruppe mit 1 bis 4, vorzugsweise 1 oder 2, C-Atomen tragen.

Aliphatische heterocyclische Reste, insbesondere solche, die sich von Stickstoffheterocyclen ableiten, können auch an einem Ringkohlenstoffatom, vorzugsweise einem dem Stickstoff enthaltenden Heteroglied benachbarten Ringkohlenstoffatom, eine Ketofunktion, ein doppelt gebundenes Sauerstoffatom, aufweisen. Dieses kann auch in seiner tautomeren Form vorliegen.

Die heterocyclischen Reste können gegebenenfalls auch an einen Benzolkern kondensiert sein, der seinerseits gegebenenfalls durch Alkyl mit 1 bis 4, vorzugsweise 1 oder 2 C-Atomen, oder Alkoxy mit 1 bis 4, vorzugsweise 1 oder 2 C-Atomen, substituiert sein kann. Beispiele für solche kondensierten Heterocyclen sind Indol und Chinazolin.

Heteroaryl ist wie oben für Het angegeben definiert, beschränkt sich allerdings auf aromatische heterocyclische Reste.

Für die erfindungsgemäße Verwendung bevorzugte Verbindungen der allgemeinen Formel I sind solche, in denen mindestens einer der Reste R¹ und R² für -CONH₂ oder -CONH-(C₁-C₆)-Alkyl steht.

Besonders bevorzugte Verbindungen der allgemeinen Formel I sind solche, in denen sowohl R¹ als auch R² für -CONH₂ oder -CONH-(C₁-C₆)-Alkyl stehen.

Weitere bevorzugte Verbindungen sind solche, in denen R¹ (C₁-C₆)-Alkyl bedeutet.

Pharmakologisch annehmbare Salze der 1.2.5-Oxadiazol-2-oxide der allgemeinen Formel I sind insbesondere Säureadditionssalze mit anorganischen oder organischen Säuren. Geeignete Säuren sind beispielsweise Chlorwasserstoff, Bromwasserstoff, Naphthalindisulfonsäuren, insbesondere Naphthalin-disulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen-oder Adipinsäure. Besonders Bevorzugt sind die Hydrochloride.

Die Herstellung der anspruchsgemäßen 1.2.5-Oxadiazol-2-oxide sowie ihrer pharmakologisch verträglichen Salze ist bekannt und beispielsweise in EP-A 38 438, EP-A 54 872 und EP-A 54 873 beschrieben.

Es ist normalerweise zweckmäßig, die anspruchsgemäßen 1.2.5-Oxadiazol-2-oxide in einer pharmazeutischen Zubereitung zu verabreichen. Dabei kommen insbesondere parenterale und topische Applikationsformen wie beispielsweise Lotionen, Cremes, Lösungen, Gele, Sprays, elastische Flüssig-Pflaster, transdermale Systeme oder Beschichtungen für Kondome in Frage.

Bevorzugt ist eine parenterale Applikation, da sie die sicherste Form ist, eine gezielte Wirkstoffmenge direkt ohne weitere Verfälschung durch andere Faktoren gezielt einzusetzen.

Zubereitungen zur parenteralen Applikation enthalten 0,1 bis 5 mg, bevorzugt 0,1 bis 2 mg Verbindung der allgemeinen Formel I pro Dosiseinheit und können in separaten Dosiseinheitsformen wie z. B. Ampullen oder Vials vorliegen. Vorzugsweise werden Lösungen des Wirkstoffes verwendet, bevorzugt wässrige Lösungen und vor allem isotonische Lösungen, aber auch Suspensionen. Diese Injektionsformen können als Fertigpräparat zur Verfügung gestellt werden oder erst direkt vor der Anwendung durch Mischen der wirksamen Verbindung, z. B. des Lyophilisats, gegebenenfalls mit weiteren festen Trägerstoffen, mit dem gewünschten Lösungs- oder Suspensionsmittel zubereitet werden. Für topische Applikationsformen ist selbstverständlich eine höhere Wirkstoffkonzentration als die für parenterale Zubereitungen genannte angezeigt.

Parenterale wie topische Formen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze zur Regelung des osmotischen Drucks oder zur Pufferung und/oder Viskositätsregulatoren enthalten.

Derartige Zusätze sind z. B. Tartrat- und Citrat-Puffer, Ethanol, Komplexbildner (wie Ethylendiamintetraessigsäure und deren nicht-toxische Salze). Zur Regelung der Viskosität kommen hochmolekulare Polymere in Frage wie beispielsweise flüssiges Polyethylenoxid, Carboxymethylcellulosen, Polyvinylpyrrolidone, Dextrane oder Gelatine. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere wie Polyethylenglykol.

Ölige Suspensionen für parenterale oder topische Anwendungen können vegetabile synthetische oder semisynthetische Öle wie beispielsweise flüssige Fettsäureester mit jeweils 8 bis 22 C-Atomen in den Fettsäureketten, z. B. Palmitin-, Laurin-, Tridecyl-, Margarin-, Stearin-, Arachin-, Myristin-, Behen-, Pentadecyl-, Linol-, Elaidin-, Brasidin-, Eruca- oder Ölsäure, die mit ein-bis dreiwertigen Alkoholen mit 1 bis 6 C-Atomen wie beispielsweise Methanol, Ethanol, Propanol, Butanol, Pentanol oder deren Isomere, Glycol oder Glycerol verestert sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfett, Kokosfettsäure-isopropylester, Ölsäureoleylester, Ölsäuredecylester, Milchsäureethylester, Dibutylphthalat, Adipinsäurediisopropylester, Polyol-Fettsäureester u. a. Ebenso geeignet sind Silikonöle verschiedener Viskosität oder Fettalkohole wie Isotridecylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden. Die genannten Stoffe haben zudem die Eigenschaften eines Spreitmittels, d. h. es erfolgt eine besonders gute Verteilung auf der Haut.

Als Lösungsmittel, Gelbildner und Lösungsvermittler kommen in Frage Wasser oder mit Wasser mischbare Lösungsmittel. Geeignet sind z. B. Alkohole wie beispielsweise Ethanol oder Isopropylalkohol, Benzylalkohol, 2-Octyldodecanol, Polyethylenglykole, Phthalate, Adipate, Propylenglykol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuran, Cyclohexanon etc.

Als Filmbildner können Celluloseether verwendet werden, die sich sowohl in Wasser als auch in organischen Lösungsmitteln lösen bzw. anquellen können und nach dem Trocknen eine Art Film bilden, wie beispielsweise Hydroxypropylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie beispielsweise Natriumcarboxymethylcellulose, Polyacrylsäure, Polymethacrylsäure und deren Salze, Natriumamylopektin-semiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure, Parfümöle.

Auch die Verwendung von Tensiden, Emulgatoren oder Netzmitteln kann zur Formulierung notwendig sein, wie z. B. von Na-Laurylsulfat, Fettalkoholethersulfaten, Di-Na-N-lauryl-β-iminodipropionat, polyoxyethyliertes Rizinusöl oder Sorbitan-Monooleat, Sorbitan-Monostearat, Cetylalkohol, Lecithin, Glycerinmonostearat, Polyoxyethylenstearat, Alkylphenolpolyglykolether, Cetyltrimethylammoniumchlorid oder Mono-/Dialkylpoly-glykolether-orthophosphorsäure-monoethanolaminsalze.

Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zur Förderung der Penetration enthalten transdermale Formulierungen vorzugsweise organische, gut hautverträgliche Lösungsmittel wie Ethanol, Methylpyrrolidon, Polyethylenglykol, Oleylalkohol, Octanol, Linolsäure, Triacetin, Propylenglykol, Glycerin, Solketal oder Dimethylsulfoxid.

Die Herstellung, Abfüllung und die Verschließung der Präparate erfolgt unter den üblichen antimikrobiellen und aseptischen Bedingungen. Auch für topischen beziehungsweise transdermalen Einsatz erfolgt eine Abpackung möglichst in separaten Dosiseinheiten zur Erleichterung der Handhabung, auch hier wie bei parenteralen Formen gegebenenfalls aus Stabilitätsgründen durch separate Abpackung der Wirkstoffe beziehungsweise deren Kombinationen als Lyophilisat, gegebenenfalls mit festen Trägerstoffen, und den erforderlichen Lösungsmitteln etc.

In einer besonderen Ausführungsform der Erfindung werden die 1.2.5-Oxadiazol-2-oxide der allgemeinen Formel I in Kombination mit einem oder mehreren synergistisch wirkenden Substanzen verwendet. Solche synergistisch wirkenden Substanzen sind beispielsweise Adenosin, Vitamine, z. B. Vitamin A oder H, Prostaglandine, z. B. E₁, Peptide wie z. B. Calcitonine gene related peptides (CGRP), Calcium-Antagonisten wie Nifedipin, Verapamil, Diltiazem, Gallopamil, Niludipin, Nimodipin, Nicardipin, Prenylamin, Fendilin, Terodilin, Nisaldipin, Nitrendipin oder Perhexilin, α-Rezeptorenblocker, beispielsweise Phentolaminmethansulfonat, Phenoxybenzamin oder Minoxidil, Relaxantien der glatten Muskulatur wie Papaverin, Trinitroglycerin, Natriumnitroprussid oder S-nitroso-N-acetylpenicillamin.

Besonders vorteilhaft und ebenfalls Gegenstand vorliegender Erfindung ist eine Einmal-Arzneimittelpackung enthaltend in steriler Abpackung eine Einmalspritze mit einer parenteralen Zubereitung eines 1.2.5-Oxadiazol-2-oxids der allgemeinen Formel I, gegebenenfalls in Kombination mit einer synergistisch wirkenden Substanz; mit Desinfektionsmitteln versehene sterile Tupfer sowie Benutzungsinformationen.

Die genannten Einmal-Fertigspritzen sind vorzugsweise vom Typ Ultrafein, wie sie beispielsweise in der Insulintherapie zur Anwendung kommen. Bevorzugt sind auch Spritzen in der Art von Autoinjektoren, die noch einfacher in ihrer Handhabung sind. Die Fertigspritzen können auch so ausgebildet sein, daß Wirkstoff und zugehöriges Injektionsmittel erst direkt vor der Injektion gemischt werden. Dies kann z.B. in Zwei-Behältnis-Ampullen bzw. -Spritzen geschehen.

Die sterilen Tupfer zur Desinfektion enthalten die üblichen Mittel zur Haut-Desinfektion wie beispielsweise Ethanol. Sie können jedoch auch noch zusätzlich erektionsfördernde Mittel in einer topisch applizier-baren Form zur Steigerung bzw. Verlängerung der Wirkung enthalten.

### Beispiel 1

Zur Herstellung einer Injektionslösung werden 500 mg 1.2.5-Oxadiazol-2-oxid-3,4-dicarbonsäure-bis-isopro-pylamid und 750 mg NaCl in einer Mischung aus Polyethylenglykol 400 und destilliertem Wasser auf 100 ml aufgefüllt und in Ampullen abgepackt.

Alternativ können anstelle des Polyethylenglykols 400 auch Propylenglykol 1.2, DMSO, Cyclodextrin oder andere gebräuchliche Lösungsvermittler eingesetzt werden. In gleicher Weise lassen sich für die Herstellung weiterer Injektionslösungen die folgenden Verbindungen verwenden:
2. 1.2.5-Oxadiazol-2-oxid-3,4-dicarbonsäure-bis-ethyl -amid
3. 1.2.5-Oxadiazol-2-oxid-3,4-dicarbonsäure-bis-methyl-amid
4. 1.2.5-Oxadiazol-2-oxid-3,4-dicarbonsäure-bis-amid
5. 1.2.5-Oxadiazol-2-oxid-3,4-dicarbonsäure-bis (2-methoxyethyl-amid)
6. 1.2.5-Oxadiazol-2-oxid-3,4-dicarbonsäure-bis-n-butylamid
7. 1.2.5-Oxadiazol-2-oxid-3,4-dicarbonsäure-bis-tert.-butylamid
8. 1.2.5-Oxadiazol-2-oxid-3,4-dicarbonsäure-bis-isobutylamid
9. 1.2.5-Oxadiazol-2-oxid-3,4-dicarbonsäure-bis-sek.-butylamid
10. 1.2.5-Oxadiazol-2-oxid-3,4-dicarbonsäure-bis-cyclopentylamid
11. 1.2.5-Oxadiazol-2-oxid-3,4-dicarbonsäure-bis-(3-methoxypropyl-amid)
12. 1.2.5-Oxadiazol-2-oxid-3,4-dicarbonsäure-bis-(2-methoxycarbonylethyl-amid)
13. 1.2.5-Oxadiazol-2-oxid-3,4-dicarbonsäure-bis-(2-ethoxyethyl-amid)
14. 1.2.5-Oxadiazol-2-oxid-3,4-dicarbonsäure-bis (ethylaminocarbonylmethyl-amid)
15. 1.2.5-Oxadiazol-2-oxid-3-methyl-4-carbonsäureamid
16. 1.2.5-Oxadiazol-2-oxid-4-methyl-3-carbonsäureamid
17. 1.2.5.-Oxadiazol-2-oxid-4-methyl-3-carbonsäuremethylamid
18. 1.2.5-Oxadiazol-2-oxid-4-phenyl-3-carbonsäure
19. 1.2.5-Oxadiazol-2-oxid-4-phenyl-3-carbonsäure-amid
20. 1.2.5-Oxadiazol-2-oxid-4-methyl-3-carbonsäureisopropylamid
21. 1.2.5.-Oxadiazol-2-oxid-4-methyl-3-carbonsäure-(2-diethylaminoethylamid)
22. 1.2.5-Oxadiazol-2-oxid-4-methyl-3-carbonsäure-(2-methoxyethylamid)
23. 4-Methyl-3-phenylsulfinyl-1.2.5-Oxadiazol-2-oxid

## Patentansprüche

1. Verwendung von 1.2.5-Oxadiazol-2-oxiden der allgemeinen Formel I 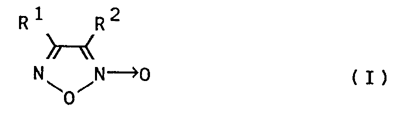 oder eines pharmakologisch verträglichen Salzes davon, wobei R¹ und R² unabhängig voneinander
a) 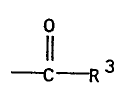 bedeuten, worin
R³ Amino, (C₁-C₆)-Alkylamino, Di-(C₁-C₆)-alkylamino, (C₄-C₇)-Cycloalkylamino, -NHNH₂, (C₆-C₁₀)-Arylamino, NH-(CH₂)ₙ-OR⁴,-NH-(CH₂)ₙCOR⁴, -NH-(CH₂)ₙCOOR⁴-, NH-(CH₂)ₙCONR⁴R^{4'}, -NH-CH(Alk)-COOR⁴, -NH-CH(Alk)-CONR⁴R⁴^{'}, -NH-(CH₂)ₙ(C₆-C₁₀)-Aryl, (C₁-C₆)-Alkyl, (C₆-C₁₀)-Aryl, Hydroxy, (C₁-C₆)-Alkoxy, (C₆-C₁₀)-Aryloxy oder (C₆-C₁₀)-Aryl(C₁-C₆)-alkyloxy ist und n eine ganze Zahl von 1 bis 4, R⁴ und R^{4'} unabhängig voneinander Wasserstoff, (C₁-C₆)-Alkyl, (C₄-C₇)-Cycloalkyl, Benzyl oder Phenethyl, Alk (C₁-C₆)-Alkyl und X -(CH₂)₂-0-(CH₂)₂-, -(CH₂)₂-N(CH₃)-(CH₂)₂-, -(CH₂)₄-oder -(CH₂)₅-bedeuten;
b) -OR⁵ bedeuten, worin
R⁵ (C₁-C₆)-Alkyl, (C₄-C₇)-Cycloalkyl, (C₂-C₇)-Alkenyl, (C₇-C₁₀)-Aralkyl, -(CH₂)ₘ -Het, oder -(CH₂)ₘ-R⁶ ist und Het für einen Heterocyclus, R⁶ für Hydroxy, (C₁-C₆)-Alkoxy, (C₁-C₆)-Alkylamino, Di-(C₁-C₆)-alkylamino oder N-(C₁-C₆)-Alkyl-N-(C₇-C₁₀)-aralkyl und m für 0, 1, 2, 3 oder 4 steht.
c) -S(O)ₚ-R⁷ bedeuten, worin
p 0,1 oder 2 und
R⁷(C₁-C₆)-Alkyl, (C₄-C₇)-Cycloalkyl, (C₆-C₁₀)-Aryl, (C₇-C₁₀)-Aralkyl, (C₂-C₇)-Alkenyl, -(CH₂)m-R⁶, -(CH₂)ₙ-CO-R⁸ oder -CH₂-CH(NHR⁹)COR⁸ ist, wobei m und R⁶ wie oben unter b) angegeben definiert sind und R⁸ Hydroxy, Amino, (C₁-C₆)-Alkyl-amino,
Di-(C₁-C₆)-alkylamino oder -OR^{7'}, wobei R^{7'} mit Ausnahme von - (CH₂)ₙ-CO-R⁸ wie R⁷ definiert ist und R⁹ Wasserstoff, (C₁-C₆)-Alkyl oder (C₂-C₇)-Alkylcarbonil bedeuten;
d) 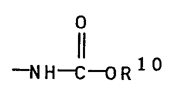 bedeuten, worin
R¹⁰ (C₁-C₆)-Alkyl, (C₆-C₁₀)-Aryl oder (C₆-C₁₀)-Aryl-(C₁-C₆)-Alkyl ist;
e) (C₁-C₆)-Alkyl, (C₄-C₇)-Cycloalkyl, (C₁-C₆)-Alkylamino, Di-(C₁-C₆)-alkylamino, (C₆-C₁₀)-Aryl, das gegebenenfalls durch (C₁-C₆)-Alkyl, (C₁-C₆)-Alkoxy, Amino, (C₁-C₆)-Alkylamino, Di-(C₁-C₆)-Alkylamino, - COO(C₁-C₆)-Alkyl, Fluor, Chlor, Brom, Nitro oder Trifluormethyl ein- , zwei- oder dreifach substituiert ist, (C₆-C₁₀)-Aryl-(C₁-C₆)-Alkyl, Heteroaryl oder -(CH₂)ₙ-COR¹¹ bedeutet, wobei n wie oben unter a) angegeben definiert ist und R¹¹ für (C₁-C₆)-Alkoxy, Amino, (C₁-C₆)-Alkylamino oder Di-(C₁-C₆)-alkylamino steht; wobei, falls einer der Reste R¹ und R² Pyridyl, Phenyl oder substituiertes Phenyl bedeutet, der andere nicht für ein Amid stehen kann und wobei, falls einer der Reste R¹ und R² Phenyl bedeutet, der andere nicht für einen der unter b) und c) genannten Reste stehen kann, zur Behandlung erektiler Dysfunktionen.

2. Verwendung gemäß Anspruch 1, dadurch gekennzeichnet, daß Verbindungen der allgemeinen Formel I eingesetzt werden, in denen mindestens einer der Reste R¹ und R² für -CONH₂ oder -CONH-(C₁-C₆)-Alkyl steht.

3. Verwendung gemäß Anspruch 1, dadurch gekennzeichnet, daß Verbindungen der allgemeinen Formel I eingesetzt werden, in denen sowohl R¹ als auch R² für -CONH₂ oder -CONH-(C₁-C₆)-Alkyl stehen.

4. Verwendung gemäß Anspruch 1, dadurch gekennzeichnet, daß Verbindungen der allgemeinen Formel I eingesetzt werden, in denen R¹ für (C₁-C₆)-Alkyl steht.

5. Verwendung gemäß einem oder mehreren der Ansprüche 1 bis 4, dadurch gekennzeichnet, daß das 1.2.5-Oxadiazol-2-oxid der allgemeinen Formel I in Kombination mit einem oder mehreren synergistisch wirkenden Substanzen eingesetzt wird.

6. Verwendung gemäß Anspruch 5, dadurch gekennzeichnet, daß die synergistisch wirkende Substanz ein Adenosin, Vitamin, Peptid, Calcium-Antagonist, α-Rezeptorenblocker oder Relaxans der glatten Muskulatur ist.

7. Einmal-Arzneimittelpackung enthaltend in steriler Abpackung
- eine Einmalspritze mit einer parenteralen Zubereitung eines 1.2.5-Oxadiazol-2-oxids der allgemeinen Formel I gemäß einem oder mehreren der Ansprüche 1 bis 4, gegebenenfalls in Kombination mit einer synergistisch wirkenden Substanz gemäß Anspruch 5 und/oder 6;
- mit Desinfektionsmitteln versehene sterile Tupfer sowie
- Benutzungsinformationen.
